# DEMANDE DE BREVET EUROPEEN

(11) **EP 1 481 661 A2**
(43) Date de publication de la demande: **01.12.2004**
(21) Numéro de dépôt: 04291243.6
(22) Date de dépôt: 14.05.2004
(51) Int. Cl.: A61K 7/02, A61K 7/48

(54) **Composition cosmétique pour le maquillage et/ou le soin de la peau, notamment du visage**

(30) Priorité: 28.05.2003 FR 0306491
(71) Demandeur: L'OREAL, 75008 Paris (FR)
(72) Inventeur: Liechty, Anne, 75017 Paris (FR); Clavel, Euriel, 75013 Paris (FR)
(74) Mandataire: Le Coupanec, Pascale

(57) **Abrégé**

La présente invention concerne une composition cosmétique de soin et/ou de maquillage de la peau et/ou des lèvres comprenant au moins une phase grasse liquide, ladite phase grasse contenant au moins un polymère filmogène lipodispersible et des particules de polyéthylène de taille moyenne en nombre inférieure ou égale à 50 µm.

## Description

La présente invention concerne une composition cosmétique pour le maquillage et/ou le soin de la peau, notamment du visage.

Cette composition peut se présenter notamment sous forme de produits coulés en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eyes liners, les compositions de protection solaire ou de coloration de la peau ou encore de maquillage du corps.

Les produits de maquillage ou de soins de la peau ou des lèvres des êtres humains comme les fonds de teint ou rouges à lèvres contiennent généralement une phase grasse à base d'huile(s) et/ou cire(s), des pigments et/ou des charges et éventuellement des additifs comme des actifs cosmétiques ou dermatologiques.

Plus particulièrement, les compositions considérées selon l'invention peuvent constituer un produit de maquillage, par exemple du visage, dont la tenue en matité du maquillage à long terme est prolongée, c'est-à-dire dont la dégradation visuelle au cours du temps est significativement réduite.

Par définition, un produit matifiant est un produit qui empêche la peau de briller et qui unifie le teint. Il est déjà connu des compositions présentant un effet matifiant. Toutefois, celles-ci ne donnent pas totalement satisfaction. Généralement, elles ne permettent pas de garantir un effet matité prolongé dans le temps et leur application nécessite d'être souvent renouvelée, notamment au-delà de quatre heures d'exposition, pour assurer l'effet attendu.

Il existe donc un besoin pour une composition cosmétique permettant d'obtenir un maquillage matifiant présentant une bonne tenue dans le temps en terme de matité.

D'une manière inattendue, les inventeurs ont constaté qu'il était possible de remédier à l'inconvénient précité en incorporant, dans la phase grasse de ce type de composition, des particules de polyéthylène en association avec au moins un composé filmogène lipodispersible.

En conséquence, la présente invention concerne selon un de ses aspects une composition cosmétique de soin et/ou de maquillage de la peau et/ou des lèvres comprenant au moins une phase grasse liquide, ladite phase grasse contenant au moins un polymère filmogène lipodispersible et des particules de polyéthylène de taille moyenne en nombre inférieure ou égale à 50 µm.

L'invention a également pour objet, selon un autre de ses aspects, un procédé de soin cosmétique et/ou de maquillage comprenant au moins une étape d'application sur la peau et/ou les lèvres d'une composition selon l'invention.

L'invention vise en outre, selon un autre de ses aspects, l'utilisation de particules de polyéthylène selon l'invention dans une composition cosmétique matifiante pour obtenir un effet de matité prolongé dans le temps. En particulier, ces particules de polyéthylène peuvent être utilisées en association avec au moins un polymère filmogène choisi parmi les polymères filmogènes liposolubles, lipodispersibles et leurs mélanges et en particulier les polymères filmogènes lipodispersibles.

### Particules de polyéthylène

Les particules de polyéthylène selon l'invention présentent une taille moyenne en nombre inférieure ou égale à 50 µm, en particulier inférieure ou égale à 30 µm, et plus particulièrement inférieure ou égale à 20 µm.

Par « taille moyenne en nombre », on désigne la dimension donnée par la distribution granulométrique statistique à la moitié de la population, dites D50.

Les particules de polyéthylène peuvent se présenter sous diverses formes et en particulier être sensiblement sphériques.

Au sens de la présente invention, sont couvertes sous le terme de particules de polyéthylène, toutes particules dérivant de l'homopolymérisation et/ou de la copolymérisation du monomère éthylène. Ces particules peuvent, le cas échéant, inclure dans leur composition des composés généralement à l'état de traces, résultant de leur procédé de préparation.

Selon une variante particulière de l'invention, les particules de polyéthylène peuvent être des particules comprenant au moins un copolymère éthylène/acide acrylique, et en particulier être constituées de copolymère(s) éthylène/acide acrylique, comme par exemple les particules vendues sous le nom commercial Flobeads® par la société SUMITOMO.

Les particules de polyéthylène peuvent, le cas échéant, être revêtues en surface, au moins partiellement, par des matières colorantes, notamment des pigments et/ou des colorants.

Les particules de polyéthylène, dans une composition selon l'invention, peuvent être présentes en une teneur variant de 0,1 à 20 % en poids, notamment de 0,5 à 15 % en poids, et en particulier de 1 à 10 % en poids par rapport au poids total de la composition.

### Polymère filmogène

La composition selon l'invention comprend au moins un polymère filmogène lipodispersible.

Ce polymère lipodispersible est généralement présent sous la forme d'une dispersion stable de particules, généralement sphériques et solides, dans la phase grasse liquide. Ces dispersions peuvent notamment se présenter sous forme de nanoparticules de polymères en dispersion. Ces nanoparticules peuvent posséder une taille allant de 5 à 600 nm, et notamment allant de 50 à 250 nm.

Les polymères des particules dispersées utilisables dans la composition selon l'invention peuvent avoir un poids moléculaire moyen en poids allant de l'ordre de 2000 à 10000000.

Le polymère peut avoir une température de transition vitreuse allant de -100°C à +300°C, notamment de -10 °C à +50 °C et plus particulièrement inférieure ou égale à environ +40 °C.

Le polymère utilisé dans la présente invention sous forme de particules dispersées dans la phase grasse peut être de toute nature. On peut ainsi employer un polymère radicalaire, un polycondensat, voire un polymère d'origine naturelle et leurs mélanges. Le polymère peut être choisi par l'homme du métier en fonction de ses propriétés.

Il s'agit plus particulièrement d'un polymère dit « filmifiable », i.e. un polymère apte à former, seul ou en association avec un agent plastifiant, un film isolable.

A titre représentatif des polymères filmifiables, on peut citer les homopolymères et les copolymères radicalaires, acryliques ou vinyliques, notamment ceux ayant une température de transition vitreuse Tg inférieure ou égale à environ +40°C et en particulier allant de -10 °C à +30 °C, et leurs mélanges.

Par « polymère radicalaire », on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, et notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tels que l'acide acrylique, l'acide méthacrylique, l'acide crotonique, l'acide maléique ou l'acide itaconique. On utilise notamment l'acide (méth)acrylique et l'acide crotonique, et plus particulièrement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, et plus particulièrement en C₁-C₈, des (méth)acrylates d'aryle, notamment d'aryle en C₆-C₁₀, des (méth)acrylates d'hydroxyalkyle, en particulier d'hydroxyalkyle en C₂-C₆ .

Parmi les (méth)acrylates d'alkyle, on peut citer le (méth)acrylate de méthyle, le (méth)acrylate d'éthyle, le (méth)acrylate de butyle, le (méth)acrylate d'isobutyle, le (méth)acrylate d'éthyl-2 hexyle et le (méth)acrylate de lauryle.

Parmi les (méth)acrylates d'hydroxyalkyle, on peut citer le (méth)acrylate d'hydroxyéthyle et le (méth)acrylate de 2-hydroxypropyle.

Parmi les (méth)acrylates d'aryle, on peut citer l'acrylate de benzyle et l'acrylate de phényle.

Les esters de l'acide (méth)acrylique particulièrement avantageux sont les (méth)acrylates d'alkyle.

Comme polymère radicalaire, on peut utiliser notamment les copolymères d'acide (méth)acrylique et de (méth)acrylate d'alkyle, en particulier d'alkyle en C₁-C₄. Plus particulièrement, on peut citer les acrylates de méthyle éventuellement copolymérisés avec l'acide acrylique tels que les copolymères de type poly(acrylate de méthyle/acide acrylique).

Comme amides des monomères acides, on peut citer les (méth)acrylamides, et notamment les N-alkyl (méth)acrylamides, en particulier d'alkyl en C₂-C₁₂ tels que le N-éthyl acrylamide, le N-t-butyl acrylamide, le N-octyl acrylamide et les N- dialkyl (C₁-C₄) (méth)acrylamides.

Les polymères vinyliques peuvent également résulter de la polymérisation de monomères à insaturation éthylénique ayant au moins un groupe amine, sous forme libre ou bien partiellement ou totalement neutralisé, ou bien encore partiellement ou totalement quaternisé. De tels monomères peuvent être par exemple choisis parmi le (méth)acrylate de diméthylaminoéthyle, le méthacrylamide de diméthylaminoéthyle, la vinylamine, la vinylpyridine et le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :
- la N-vinylpyrrolidone; la vinylcaprolactame; les vinyl N-alkyl(C₁-C₆) pyrroles; les vinyl-oxazoles; les vinyl-thiazoles; les vinylpyrimidines; les vinylimidazoles ; et
- les oléfines tels que l'éthylène, le propylène, le butylène, l'isoprène et le butadiène.

Le polymère vinylique peut être réticulé à l'aide de monomère difonctionnel, notamment comprenant au moins deux insaturations éthyléniques, tel que le diméthacrylate d'éthylène glycol et le phtalate de diallyle.

De façon non limitative, les polymères de l'invention peuvent être choisis parmi les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; copolymères acryliques-silicone ; polyacrylamides ; polymères siliconés, polymères fluorés ; et leurs mélanges.

Selon une variante particulière de l'invention, les polymères lipodispersibles peuvent être stabilisés en surface par au moins un polymère liposoluble par exemple tel que ceux décrits ci-après ou ceux décrits dans la demande de brevet EP 1 002 528.

La dispersion de polymère filmogène peut être préparée comme décrit dans le document EP-A-749747 ou dans le document EP 1 002 528. Plus précisément, la polymérisation est effectuée en dispersion, c'est-à-dire par précipitation du polymère en cours de formation, avec protection des particules formées avec un stabilisant. Des stabilisants sont notamment décrits dans EP 749 747.

Selon une variante de l'invention, ce polymère lipodispersible peut être associé à au moins un polymère liposoluble.

Les polymères liposolubles peuvent être de toute nature chimique et englobent notamment :

a) les homopolymères et les copolymères liposolubles et amorphes d'oléfines, de cyclo-oléfines, de butadiène, d'isoprène, de styrène, d'éthers, d'esters ou d'amides vinyliques, d'esters ou d'amides d'acide (méth)acrylique comprenant un groupement alkyl en C₄-C₅₀ linéaire, ramifié ou cyclique. Les homopolymères et les copolymères liposolubles peuvent être choisis parmi ceux obtenus à partir des monomères choisis dans le groupe constitué par le (méth)acrylate d'isooctyle, le (méth)acrylate d'isononyle, le 2-éthylhexyl (méth)acrylate, le (méth)acrylate de lauryle, le (méth)acrylate d'isopentyle, le (méth)acrylate de n-butyle, le (méth)acrylate d'isobutyle, le (méth)acrylate de tertio-butyle, le (méth)acrylate de tridécyle, le (méth)acrylate de stéaryle ou leurs mélanges. On citera par exemple le copolymère acrylate d'alkyle/ acrylate de cycloalkyle vendu par PHOENIX CHEM. sous la dénomination GIOVAREZ AC-5099 ML.

Comme polymères filmogènes liposolubles, on peut également citer les copolymères de la vinyl pyrrolidone (VP) et notamment les copolymères de la vinylpyrrolidone et d'alcène en C₂-C₄₀, et en particulier en C₃ à C₂₀. A titre d'exemple de copolymère de VP utilisable dans l'invention, on peut citer les copolymères de VP/acétate vinyle, VP/méthacrylate d'éthyle, VP/méthacrylate d'éthyle/acide méthacrylique, VP/eicosène, VP/hexadécène, VP/triacontène, VP/styrène, VP/acide acrylique/méthacrylate de lauryle et la polyvinylpyrrolidone (PVP) butylée.

Comme copolymères liposolubles particuliers, on peut également citer :
i) les polymères greffés acrylique-silicone à squelette silicone et greffons acryliques ou à squelette acrylique et greffons silicones, tels que les produit vendu sous la dénomination "SA 70.5", "SA 70", "VS 80" par 3M et notamment décrits dans les brevets US 5 725 882, US 5 209 924, US 4 972 037, US 4 981 903, US 4 981 902, US 5 468 477, US 5 219 560, EP 0 388 582, US 5032460 ou WO 93/23009,
ii) les polymères liposolubles appartenant à l'une des classes décrites ci-dessus et porteurs de groupes fluorés, en particulier ceux décrits dans le brevet US 5 948 393 et les copolymères (méth)acrylate d'alkyle / (méth)acrylate de perfluoroalkyle décrits dans les brevets EP 0 815 836 et US 5 849 318,
iii) les polymères ou copolymères résultant de la polymérisation ou copolymérisation d'un monomère éthylénique, comportant une ou plusieurs liaisons éthyléniques, de préférence conjuguées (ou diène). Ce ou ces agents sont en particulier des copolymères vinyliques, acryliques ou méthacryliques qui peuvent être séquencés et notamment de type dibloc ou tribloc, voire même de type multibloc ou en étoile.

Le polymère filmogène éthylénique peut comprendre notamment un bloc styrénique (S), un bloc alkylstyrénique (AS), un bloc éthylène/butylène (EB), un bloc éthylène/propylène (EP), un bloc butadiène (B), un bloc isoprène (I), un bloc acrylate (A), un bloc méthacrylate (MA) ou une association de ces blocs.

En particulier, on peut utiliser comme polymère filmogène un copolymère comportant au moins un bloc styrénique. Tout particulièrement, on peut utiliser un copolymère tribloc et en particulier ceux du type polystyrène/polyisoprène ou polystyrène/polybutadiène tels que ceux vendus sous le nom de 'LUVITOL HSB' par BASF et ceux du type polystyrène/copoly(éthylène-propylène) ou encore du type polystyrène/copoly(éthylène-butylène) tels que ceux vendus sous la marque 'KRATON' par Shell Chemical Co ou de Gelled Permethyl 99A par Penreco. On peut aussi utiliser des copolymères styrène-méthacrylate.

Comme polymère filmogène utilisable dans la composition de l'invention, on peut citer également, par exemple, les Kraton G1650 (SEBS), Kraton G1651 (SEBS), Kraton G1652 (SEBS), Kraton G1657X (SEBS), Kraton G1701X (SEP), Kraton G1702X (SEP), Kraton G1726X (SEB), Kraton G1750X (EP) multibras, Kraton G1765X (EP) multibras, Kraton D-1101 (SBS), Kraton D-1102 (SBS), Kraton D-1107 (SiS), les Gelled Permethyl 99A -750, Gelled Permethyl 99A- 753-58 (mélange de tribloc et de polymère bloc en étoile), Gelled Permethyl 99A- 753-59 (mélange de tribloc et de polymère bloc en étoile), Versagel 5970 et Versagel 5960 de chez Penreco (mélange de tribloc et de polymère en étoile dans isododécane), OS 129880, OS 129881 et OS 84383 de chez Lubrizol (copolymère styrène-méthacrylate).

b) les polycondensats liposolubles, amorphes, en particulier ceux ne comportant pas de groupes donneurs d'interaction hydrogène, notamment des polyesters à chaînes latérales alkyles en C₄-C₅₀ ou bien les polyesters résultant de la condensation d'acides gras dimères, ou encore les polyesters comportant un segment silicone sous forme de séquence, greffon, ou groupe terminal, solides à température ambiante, par exemple tels que définis dans la demande de brevet FR-A-2831430,

c) les polysaccharides liposolubles et amorphes comportant des chaînes latérales alkyles (éthers ou esters), en particulier les alkyl-celluloses comprenant un radical alkyle linéaire ou ramifié, saturé ou insaturé, en C₁-C₈ tels que l'éthylcellulose et la propylcellulose.

D'une façon générale, les polymères liposolubles filmogènes de l'invention peuvent avoir un poids moléculaire moyen en poids variant de 1.000 à 500.000, notamment de 2.000 à 250.000, et une température de transition vitreuse allant de -100 °C à +300 °C, notamment de -50 °C à +100 °C, et en particulier de -10 °C à +90 °C.

D'une manière générale, la composition selon l'invention peut comprendre de 0,1 % à 25 % en poids, notamment de 1 % à 20 % en poids, et en particulier de 5 % à 16 % en poids de polymère filmogène, notamment de polymère filmogène lipodispersible (exprimé en matière sèche), par rapport au poids total de la composition.

La composition selon l'invention peut comprendre au moins un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Les agents de coalescence ou plastifiants utilisables dans l'invention sont notamment ceux cités dans le document FR-A-2 782 917.

### Phase grasse liquide

La phase grasse liquide de la composition peut comprendre une ou plusieurs huiles cosmétiquement ou dermatologiquement acceptables, et de façon générale physiologiquement acceptables.

Ainsi, elle peut comprendre au moins une huile, pouvant notamment être choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Par "phase grasse liquide", on entend tout milieu non aqueux liquide à température ambiante (25 °C) et pression atmosphérique. Cette phase grasse peut contenir une phase grasse liquide volatile et/ou une phase grasse non volatile.

Par "phase grasse volatile", on entend tout milieu non aqueux susceptible de s'évaporer de la peau ou des lèvres, en moins d'une heure. Cette phase volatile comporte notamment des huiles ayant une pression de vapeur, à température ambiante et pression atmosphérique allant de 10⁻³ à 300 mm de Hg (0,13 Pa à 40 000 Pa).

La phase grasse liquide selon l'invention, peut comprendre au moins un composé choisi parmi les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxanes (PDMS), éventuellement phénylées telles que les phényltriméthicones ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées et les huiles perfluorées.

Avantageusement, on peut utiliser une ou plusieurs huiles volatiles à température ambiante.

Elles peuvent être hydrocarbonées ou siliconées et comporter éventuellement des groupements alkyle ou alkoxy, pendants ou en bout de chaîne siliconée.

Comme huile volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone, tels que notamment l'octaméthylcyclotétrasiloxane, le décaméthylcyclopentasiloxane, l'hexadécaméthylcyclohexasiloxane, l'heptaméthylhexyltrisiloxane et l'heptaméthyloctyltrisiloxane, ainsi que les isoparaffines en C₈-C₁₆ telles que les ISOPARs,® les PERMETYLs® et notamment l'isododécane.

La phase grasse liquide totale de la composition peut représenter de 5 % à 97 % en poids et notamment de 20 à 85 % en poids par rapport au poids total de la composition. La partie non volatile peut représenter de 0,1 à 80 % en poids et notamment de 1 à 50 % en poids par rapport au poids total de la composition.

### Corps gras solides

La composition peut comprendre en outre au moins un corps gras pâteux ou solide à température ambiante choisi par exemple parmi les cires, les gommes et leurs mélanges.

Par « cire », au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en rétablissant la température du mélange à la température ambiante, on obtient une recristallisation de la cire dans les huiles du mélange.

La dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée, divisée par la surface du cylindre du texturomètre en contact avec la cire.

Les cires, au sens de la présente demande, peuvent être hydrocarbonées, siliconées et/ou fluorées et comporter éventuellement des fonctions ester ou hydroxyle. Elles peuvent être notamment d'origine naturelle ou synthétique. A titre illustratif et non limitatif des cires, on peut notamment citer la cire d'abeilles éventuellement modifiée, la cire de Carnauba, de Candellila, d'Ouricoury, du Japon, de fibres de liège ou de canne à sucre, la cérésine, les cires de paraffine, de lignite, les cires microcristallines, la cire de lanoline, la cire de Montan, les ozokérites, les huiles hydrogénées comme l'huile de jojoba hydrogénée ou copolymérisation de l'éthylène, les cires obtenues par synthèse de Fischer-Tropsch, les esters d'acides gras et les glycérides concrets à 45 °C, les cires de silicone comme les alkyle, alcoxy et/ou esters de poly(di)méthylsiloxane solide à 45 °C, ayant de 10 à 45 atomes de carbone, certains acides gras comme l'acide stéarique, myristique ou béhénique, et leurs mélanges.

La composition selon l'invention peut comprendre au moins une cire en une teneur variant de 0,1 à 20 % en poids, notamment de 2 à 15 % en poids, et en particulier de 3 à 12 % en poids par rapport au poids total de la composition.

### Phase aqueuse

La composition selon l'invention peut comprendre également une phase aqueuse contenant de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants autres que l'eau comme par exemple les alcools primaires tels que l'éthanol et l'isopropanol, les glycols tels que le glycérol, le propylène glycol, le butylène glycol, le dipropylène glycol, le diéthylène glycol, les éthers de glycol tel que les alkyl (C₁-C₄) éther de mono, di- ou tripropylène glycol, mono, di- ou triéthylène glycol, et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

La phase aqueuse, et notamment l'eau, peut être notamment présente dans la composition selon l'invention en une teneur allant de 2 à 75 %, en poids, et en particulier de 5 à 50 % en poids, par rapport au poids total de la composition.

La composition peut se présenter sous forme d'émulsion huile-dans-eau (H/E) ou eau-dans-huile (E/H). Elle peut se présenter également sous forme anhydre.

Lorsque la composition selon l'invention est sous forme d'émulsion, elle peut également comprendre un tensioactif ou un mélange de tensioactifs dont le HLB (balance hydrophile/lipophile) est généralement adapté à la nature de l'émulsion à stabiliser.

Comme tensioactif utilisable dans l'invention, adapté à l'obtention d'une émulsion E/H on peut citer ceux ayant un HLB inférieur 7 et notamment les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone. Comme tensioactif utilisable dans l'invention pour l'obtention d'une émulsion H/E on peut citer ceux ayant un HLB supérieur à 7 comme les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés et les diméthicones copolyols. De façon générale, on peut utiliser tout tensioactif ionique (cationique ou anionique) amphotère et tout tensioactif non ionique, bien connus de l'homme du métier.

Le tensioactif peut être présent dans la composition en une teneur allant de 0,3 à 10 % en poids, et notamment de 1 à 5 % en poids par rapport au poids total de la composition.

La composition selon l'invention peut comprendre en outre au moins un composé pulvérulent annexe, c'est-à-dire différent des polyéthylènes considérés selon l'invention. Le composé pulvérulent annexe peut être choisi parmi les charges, les matières colorantes pulvérulentes, telles que les pigments et les nacres, et leurs mélanges.

Par « charges », il faut comprendre des particules incolores ou blanches, minérales ou de synthèse, lamellaires ou non lamellaires.

Ces charges peuvent être choisies parmi le talc, le mica, la silice, le kaolin, les poudres de poly-β-alanine, la lauroyl-lysine, l'amidon, le nitrure de bore, les microsphères creuses polymériques telles que celles de chlorure de polyvinylidène/acrylonitrile telles que l'Expancel® (Nobel Industrie), les particules de polymère acrylique, notamment de copolymère d'acide acrylique comme le Polytrap® (Dow Corning), les poudres de polyuréthane, les microbilles de résine de silicone (Tospearls® de Toshiba, par exemple), le carbonate de calcium précipité, le dicalcium phosphate, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et leurs mélanges.

Ces charges peuvent être traitées ou non en surface notamment pour les rendre lipophiles.

Ces charges peuvent être présentes dans la composition en une teneur allant de 0,1 à 30 % en poids, et notamment de 0,1 à 10 % en poids par rapport au poids total de la composition.

Par « pigments », on entend désigner, au sens de la présente invention, des particules blanches ou colorées, minérales ou organiques, insolubles dans la phase organique liquide, destinées à colorer et/ou opacifier la composition.

Les pigments peuvent être des pigments minéraux et/ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

On utilise en particulier des pigments d'oxydes de fer ou de dioxyde de titane.

Les pigments peuvent être traités, le cas échéant, avec un agent hydrophobe pour les rendre compatible avec la phase grasse de la composition.

De tels pigments traités hydrophobes sont notamment décrits dans la demande EP-A-1086683.

Par « nacres », on entend désigner, au sens de la présente invention, des particules irisées, notamment produites par certains mollusques dans leur coquille ou bien synthétisées, insolubles dans le milieu de la composition.

Les matières colorantes pulvérulentes peuvent être présents en une teneur allant de 0,5 à 30 % en poids, notamment allant de 5 à 20 % en poids, et en particulier allant de 6 à 15 % en poids, par rapport au poids total de la composition.

La composition peut comprendre en outre au moins un colorant lipophile et/ou au moins un colorant hydrophile.

Par « colorant », on entend désigner, au sens de la présente invention, des composés généralement organiques solubles dans les corps gras comme les huiles ou dans une phase hydroalcoolique.

Parmi les colorants liposolubles utilisables selon l'invention, on peut citer le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, et les bromoacides.

La composition selon l'invention peut contenir en outre un ou plusieurs des adjuvants habituels dans le domaine cosmétique tels que des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; et leurs mélanges.

Comme actifs utilisables dans la composition selon l'invention, on peut citer par exemple les agents hydratants tels que les hydrolysats de protéines et les polyols comme la glycérine, les glycols comme les polyéthylène glycols, et les dérivés de sucre ; les extraits naturels ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les enzymes ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; et leurs mélanges.

La composition selon l'invention peut en outre comprendre au moins un filtre solaire (ou filtres U.V.). Celui-ci peut être choisi parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.

Bien entendu, l'homme de l'art veillera à choisir le ou les éventuels adjuvants ajoutés à la composition selon l'invention et à ajuster leurs quantités de manière telle que les propriétés avantageuses attachées intrinsèquement à la composition conforme à l'invention ne soient pas, ou substantiellement pas, altérées par l'addition envisagée.

Les exemples suivants sont donnés à titre illustratif et sans caractère limitatif de l'invention.

### Exemple 1 : Préparation d'une dispersion de polymère filmogène lipodispersible

On prépare une dispersion de copolymère non réticulé d'acrylate de méthyle et d'acide acrylique dans un rapport 85/15, dans de l'isododécane, selon la méthode de l'exemple 7 du document EP-A-749 747. On obtient ainsi une dispersion de particules de poly(acrylate de méthyle/acide acrylique) stabilisées en surface dans de l'isododécane par un copolymère dibloc séquencé polystyrène/copoly(éthylène-propylène) vendu sous le nom de KRATON G1701 (Shell), ayant un taux de matière sèche de 22,6 % en poids et une taille moyenne des particules de 175 nm (polydispersité : 0,05) et une Tg de 20 °C. Ce copolymère est filmifiable à température ambiante (25 °C).

### Exemple 2

On a préparé un fond de teint sous forme d'émulsion eau-dans-huile ayant la composition suivante :

### Phase huileuse :

- Dispersion de polymère filmogène lipodispersible de l'exemple 1 55,52 g
- Isododécane gélifié par Kraton (tribloc et radial) vendu sous la dénomination commerciale Versagel M5950 par la société Penreco 4,48 g
- Isododécane 1,97 g
- Mélange de polydiméthylsiloxane à groupements oxyéthyléne oxypropyléne α-ω (DP : :100-58/42) et de cyclopentasiloxane (85/15) vendu sous la dénomination commerciale "Abil EM 97" par la Société Goldschmidt 0,90 g
- Cyclopenta diméthylsiloxane 4,00 g
- Poudre de Nylon 12 vendue sous la dénomination commerciale Orgasol 2002 Extra D Nat. Cos. par la société Atochem 3,00 g
- Poudre de copolymère éthylène/acrylique (Granulométrie : 10 µm) vendus sous la dénomination Flobeads® EA-209 par la société Sumitomo 4,00 g
- Mélange d'hectorite distéardimonium, de cyclopentasiloxane et d'éthanol (10/85/5) (Bentone gel V5-5V de Elementis Specialities) 3,53 g
- Mono-di-glycérides d'acide iso-stéarique estérifiés par de l'acide succinique, non stabilisés, vendus sous la dénomination commerciale « Inwitor 780 K » par la Société Sasol 0,30 g
- Oxydes de fer enrobés de stéaroylglutamate d'aluminium 3,21 g
- Oxyde de titane enrobé de stéaroylglutamate d'aluminium 6,79 g

### Phase aqueuse :

- Glycérine 3,00 g
- Sulfate de magnésium 0,70 g
- Conservateurs qs
- Eau qsp 100,00 g

L'émulsion est préparée en mélangeant à température ambiante les constituants de la phase huileuse puis en ajoutant la phase aqueuse préalablement préparée, sous agitation.

Ce fond de teint s'applique facilement sur la peau avec une bonne sensation d'onctuosité au toucher, et le maquillage obtenu procure un dépôt doux, la matité au moment de l'application est satisfaisante et persiste bien au-delà de quatre heures.

## Revendications

1. Composition cosmétique de soin et/ou de maquillage de la peau et/ou des lèvres comprenant au moins une phase grasse liquide, ladite phase grasse contenant au moins un polymère filmogène lipodispersible et des particules de polyéthylène de taille moyenne en nombre inférieure ou égale à 50 µm.

2. Composition selon la revendication 1, **caractérisée en ce que** les particules de polyéthylène possèdent une taille moyenne en nombre inférieure ou égale à 30 µm et notamment inférieure ou égale à 20 µm.

3. Composition selon la revendication 1 ou 2, **caractérisée en ce que** les particules de polyéthylène comprennent au moins un copolymère éthylène/acide acrylique et notamment sont constituées de copolymère(s) éthylène/acide acrylique.

4. Composition selon l'une quelconque des revendications 1 à 3, **caractérisée en ce qu'**elle comprend de 0,1 à 20 % en poids, notamment de 0,5 à 15 % en poids, et en particulier de 1 à 10 % en poids de particules de polyéthylène, par rapport au poids total de la composition.

5. Composition selon l'une quelconque des revendications 1 à 4, **caractérisée en ce que** le polymère filmogène lipodispersible est choisi parmi les polyuréthannes, polyuréthannes-acryliques, polyurées, polyurée/polyuréthannes, polyester-polyuréthannes, polyéther-polyuréthannes, polyesters, polyesters amides, polyesters à chaîne grasse, alkydes, polymères ou copolymères acryliques et/ou vinyliques, copolymères acryliques-silicone, polyacrylamides, polymères siliconés, polymères fluorés et leurs mélanges.

6. Composition selon l'une quelconque des revendications 1 à 5, **caractérisée en ce que** le polymère filmogène lipodispersible est choisi parmi les homopolymères et les copolymères radicalaires, acryliques ou vinyliques, notamment ayant une température de transition vitreuse (Tg) inférieure ou égale à +40 °C et en particulier allant de -10 à +30 °C, et leurs mélanges.

7. Composition selon l'une quelconque des revendications 1 à 6, **caractérisée en ce que** le polymère lipodispersible est un polymère acrylique de type poly(acrylate de méthyle /acide acrylique).

8. Composition selon l'une quelconque des revendications 1 à 7, **caractérisée en ce que** ledit polymère filmogène lipodispersible est stabilisé en surface par au moins un polymère filmogène liposoluble.

9. Composition selon l'une quelconque des revendications 1 à 8, **caractérisée en ce qu'**elle comprend en outre au moins un polymère filmogène liposoluble.

10. Composition selon la revendication 8 ou 9, **caractérisée en ce que** le polymère filmogène liposoluble est choisi parmi les homopolymères et copolymères liposoluble et amorphes d'oléfines, de cyclo oléfines, de butadiène, d'isoprène, de styrène, d'éthers, d'esters ou d'amides vinyliques, d'esters ou d'amides d'acide (méth)acrylique comprenant un groupement alkyle en C₄₋₅₀ linéaire, ramifié ou cyclique ; les polycondensats liposolubles amorphes, notamment des polyesters à chaînes latérales alkyles en C₄₋₅₀ ou bien les polyesters résultant de la condensation d'acides gras dimères ; les polysaccharides liposolubles et amorphes comportant des chaînes latérales alkyles (éthers ou esters) ; les copolymères de la vinylpyrrolidone (VP) ; les polymères greffés acrylique silicone à squelette acrylique et greffons silicones ou à squelette silicone et greffons acrylique ; et leurs mélanges.

11. Composition selon la revendication 8, 9 ou 10, **caractérisée en ce que** le polymère filmogène liposoluble comprend au moins un copolymère tribloc et en particulier un copolymère du type polystyrène/polyisoprène ou polystyrène/polybutadiène ou encore du type polystyrène/copoly(éthylène butylène) ou polystyrène copoly(éthylène propylène).

12. Composition selon l'une quelconque des revendications 1 à 11, **caractérisée en ce qu'**elle comprend de 0,1 à 25 % en poids, notamment de 1 à 20 % en poids, et en particulier de 5 à 16 % en poids d'au moins un polymère filmogène (exprimé en matière sèche) par rapport au poids total de la composition.

13. Composition selon l'une quelconque des revendications 1 à 12, **caractérisée en ce que** la phase grasse liquide comprend au moins une huile choisie parmi les huiles d'origine végétale, minérale, animale, de synthèse, carbonées, hydrocarbonées, fluorées et/ou siliconées, et leurs mélanges.

14. Composition selon l'une quelconque des revendications 1 à 13, **caractérisée en ce que** la phase grasse liquide comprend au moins un composé choisi parmi l'huile de paraffine, de vaseline, de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorés, les huiles perfluorées ; les huiles volatiles telles que l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en C₈-C₁₆, et l'isododécane.

15. Composition selon l'une quelconque des revendications 1 à 14, **caractérisée en ce que** la phase grasse liquide est présente en une teneur variant de 5 à 97 % en poids, et notamment de 20 à 85 % en poids par rapport au poids total de la composition.

16. Composition selon l'une quelconque des revendications 1 à 15, **caractérisée en ce qu'**elle comprend en outre au moins un corps gras pâteux ou solide à température ambiante choisi parmi les cires, les gommes et leurs mélanges.

17. Composition selon l'une quelconque des revendications 1 à 16, **caractérisée en ce qu'**elle se présente sous la forme d'une émulsion eau-dans-huile ou huile-dans-eau.

18. Composition selon l'une quelconque des revendications 1 à 16, **caractérisée en ce qu'**elle est anhydre.

19. Composition selon l'une quelconque des revendications 1 à 18, **caractérisée en ce qu'**elle comprend au moins un composé pulvérulent annexe choisi parmi les charges, les pigments, les nacres et leurs mélanges.

20. Composition selon l'une quelconque des revendications 1 à 19, **caractérisée en ce qu'**elle se présente sous la forme d'une composition de maquillage.

21. Composition selon l'une quelconque des revendications 1 à 20, **caractérisée en ce qu'**elle se présente sous la forme d'un fond de teint, d'un produit anti-cernes, d'un fard à paupières ou à joues, d'un eye-liner, d'une composition de protection solaire ou de coloration de la peau ou d'une composition de maquillage du corps.

22. Composition selon la revendication 21, **caractérisée en ce qu'**il s'agit d'un fond de teint.

23. Procédé de soin cosmétique et/ou de maquillage de la peau et/ou des lèvres comprenant au moins une étape d'application sur la peau et/ou les lèvres d'une composition selon l'une quelconque des revendications 1 à 22.

24. Utilisation de particules de polyéthylène telles que définies selon l'une quelconque des revendications 1 à 4 dans une composition cosmétique matifiante pour obtenir un effet de matité prolongé dans le temps.

25. Utilisation selon la revendication 24, avec lesdites particules de polyéthylène étant associées à au moins un polymère filmogène choisi parmi les polymères filmogènes liposolubles, lipodispersible et leurs mélanges.
